(19) Europäisches Patentamt
European Patent Office
Office européen des brevets

(11) Publication number: **0 410 244 A1**

# (12) EUROPEAN PATENT APPLICATION

(21) Application number: **90113516.0**

(22) Date of filing: **14.07.90**

(51) Int. Cl.5: **C07C 59/68**, C07C 69/736, C07C 317/46, C07C 323/54, C07C 235/28, A61K 31/20, A61K 31/22, A61K 31/16

(30) Priority: **27.07.89 US 387279**

(43) Date of publication of application: **30.01.91 Bulletin 91/05**

(84) Designated Contracting States: **AT BE CH DE DK ES FR GB GR IT LI LU NL SE**

(71) Applicant: **BAYER AG**
**D-5090 Leverkusen 1 Bayerwerk(DE)**

(72) Inventor: **Abram, Trevor S.**
**214 Marlow Bottom Road**
**Marlow, Buckinghamshire SL7 3PR(GB)**
Inventor: **Biddlecom, William G.**
**3220 E. Jackson Boulevard**
**Elkhart, Indiana 46516(US)**
Inventor: **Jennings, Michael A.**
**42 Lynhurst Crescent**
**Hillingdon, Middlesex, UB10 9EG(GB)**
Inventor: **Norman, Peter**
**4 St. Andrews Way, Cippenham**
**Slough, Berkshire, SL1 5NX(GB)**
Inventor: **Tudhope, Stephen R.**
**47 Kentons Lane**
**Windsor, Berkshire, SL4 4JH(GB)**

## (54) Leukotriene antagonists.

(57) Leukotriene antagonists are disclosed comprising compounds having the general formula represented by the formula shown below, esters, amides and physiologically acceptable salts thereof. These compounds retain the natural 5(S) 6(R) configuration, the 5-hydroxyl group and the natural unsaturated tail with the peptide replaced by a substituted aromatic nucleus linked via a sulfur or oxygen substituent linking a substituent to the 6 position.

EP 0 410 244 A1

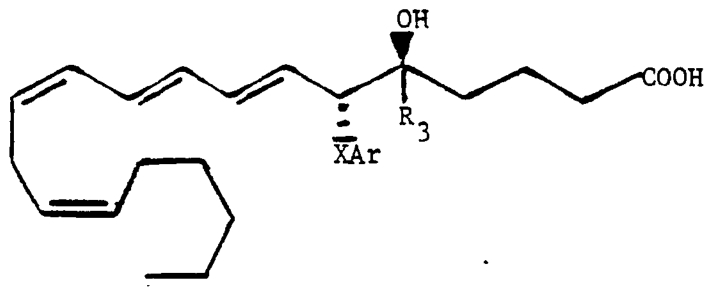

I

# LEUKOTRIENE ANTAGONISTS

## Field of the Invention

The invention provides leukotriene antagonists based on modifications of the peptide and head portions of naturally occurring leukotrienes.

## Utility

Leukotrienes, like prostaglandins, are metabolites of the polyunsaturated essential fatty acids. Leukotrienes are generated via the lipoxygenase enzymatic pathway:

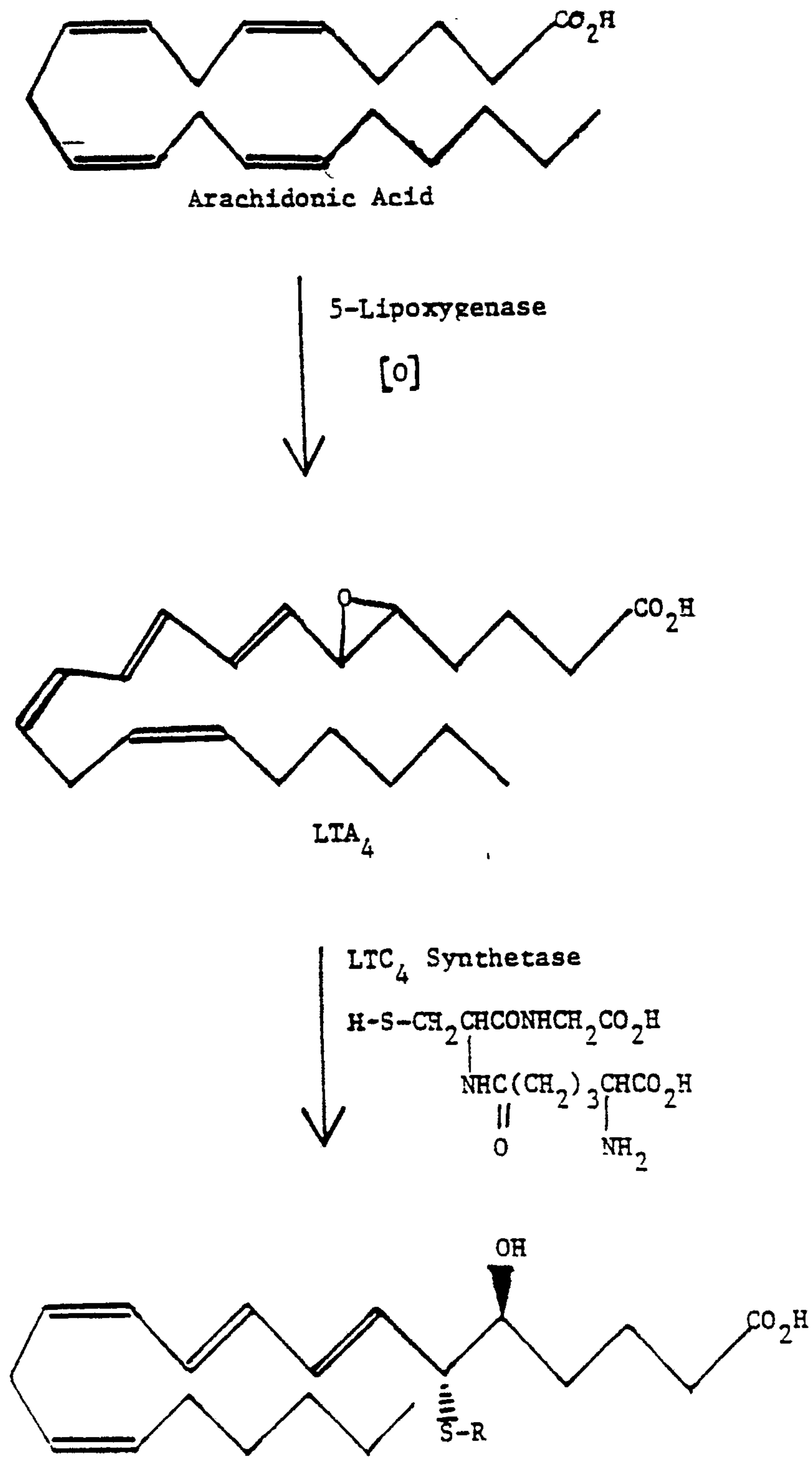


$LTC_4$ R = cys-C-gly-N-γ-glu

$LTD_4$ R = cys-C-gly

$LTE_4$ R = cys

The intermediate $LTA_4$ is a transient species which can be converted in vivo into the peptidoleukotriene $LTC_4$ by attack of the sulfhydryl group of glutathione on the epoxide ring. $LTC_4$ is converted to $LTD_4$ and $LTE_4$ by the sequential action of peptidases.

The peptidoleukotrienes, such as those shown above, have potent actions on pulmonary, vascular and

gastro-intestinal smooth muscle. In particular the leukotrienes display bronchoconstrictor effects and are believed to play a role in the pathophysiology of asthma. Since bronchoconstrictor effects are considered to be mediated by specific receptors in the lung, one approach to treatment of asthma could be the development of agents which would antagonize the actions of leukotrienes at these receptors. The leukotriene antagonists of this invention can be used to treat asthma since they antagonize the actions of leukotrienes in the lungs and block the bronchoconstrictor effects.

Conceptually the leukotriene molecule may be considered as having three areas amenable to modifi cation: (1) Head (2) Peptide (3) Tail. These areas are depicted below.

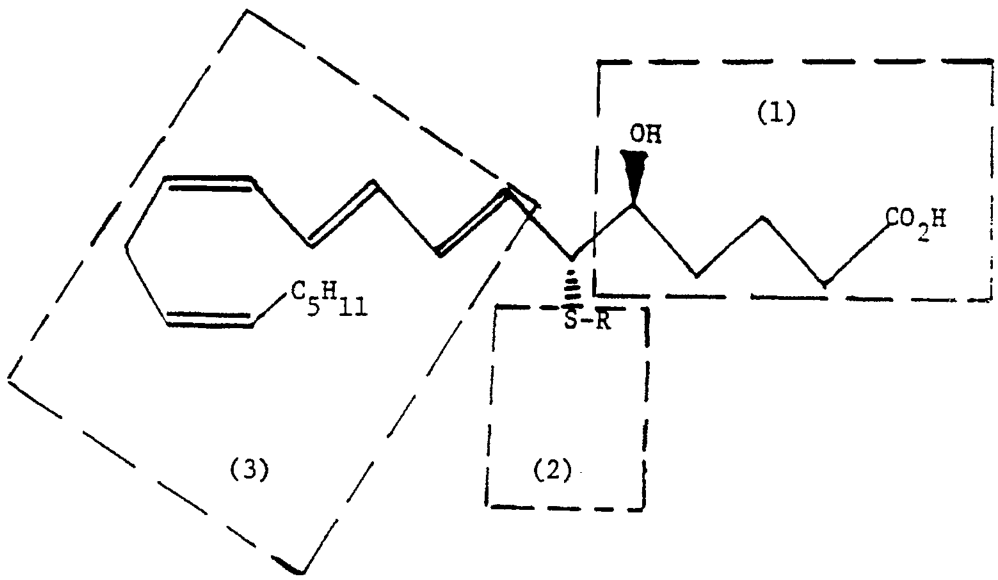

To produce leukotriene antagonists, efforts have been made to modify each of these areas.

In U.S. Patent No. 4,513,005, to Eli Lilly, many modifications to both the peptide and tail portions of the molecule are suggested.

Working with the basic structure,

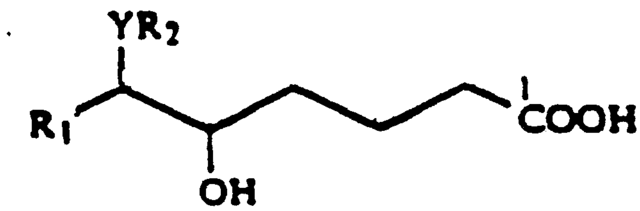

(A)

three groups of variations containing many possible combinations are suggested for $R_1$, the "tail" portion. In one part $R_1$ can be an aliphatic , saturated or unsaturated hydrocarbon radical of up to 20 carbon atoms , unsubstituted or substituted by at least one substituent selected from halogen, hydroxy, $C_{3-6}$ alkoxy, $C_{3-6}$ cycloalkyl, aryl or heteroaryl, the cycloalkyl or heteroaryl being unsubstituted or substituted by at least one subtituent selected from hydroxy, halogen and alkyl, alkenyl or alkynyl of up to 10 carbon atoms (emphasis added). The added emphasis indicates the selection required to obtain the 'natural' tail found in leukotrienes themselves.

Y is defined as -S-, -SO- or $-SO_2-$ and $R_2$ is defined as two groups of variations including:

aryl or heteroaryl, unsubstituted or substituted by one or more substituents selected from $C_{1-4}$ alkyl, $C_{1-4}$ alkoxy $C_{2-5}$ acyl, halogen, hydroxy , carboxy , nitro, trihalomethyl, phenyl $C_{1-4}$ acylamino and $NHR_4$. (Emphasis is added to indicate substituents relevant to this invention.)

Obviously a vast number of permutations are possible with the substituents above. Only example 14

discloses a compound having the natural tail (tetradecatetraenyl) with an aryl group as $R_2$. In the compound shown, $R_2$ is a benzyl group.

Preferred compounds have $R_1$ as substituted, or unsubstituted, phenyl or naphthyl and $R_2$ as substituted, or unsubstituted, phenyl. Another preferred compound has $R_1$ as PhCH=CH or $PhCH_2CH_2$ and $R_2$ may be substituted, or unsubstituted, phenyl. These compounds are stated to have an $IC_{50}$ $<10^{-4}$M against the "Slow Reacting Substance of Anaphylaxis" (SRS-A) on guinea-pig ileum.

EP 123543 to Merck Frost claims a very wide range of compounds with a highly complex general formula.

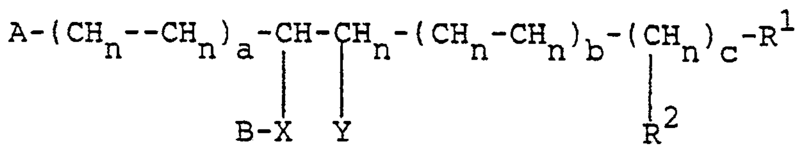

This formula has been rewritten below to show its resemblance to the Lilly representation (A). The major difference is that this patent claims a wider range of $R_1$.

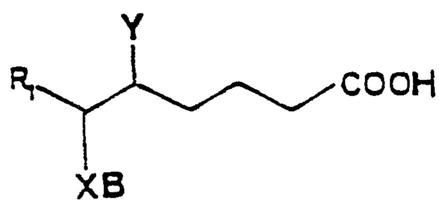

(B)

Although the definition of $R_1$ is very wide ranging it is defined in such a manner that the natural form of the tetradecatetraenyl moiety, i.e. the natural tail, is not included. In this application X can be oxygen, -O- in addition to S, SO and $SO_2$ and B may be a substituted phenyl ring or various heterocycles. However, the definition of B does not include naphthyl, pyridyl or pyrimidyl. Although B does not include naphthalene by definition, such a compound is disclosed (example 79). The EP '543 patent claims two p-substituted benzoic acids as shown below.

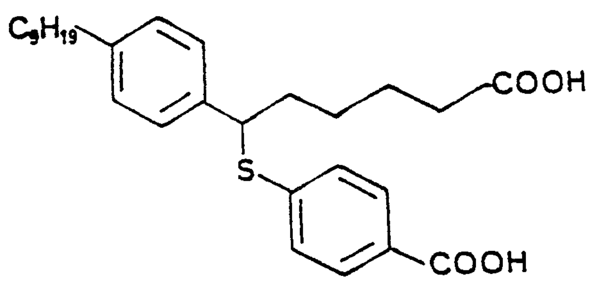

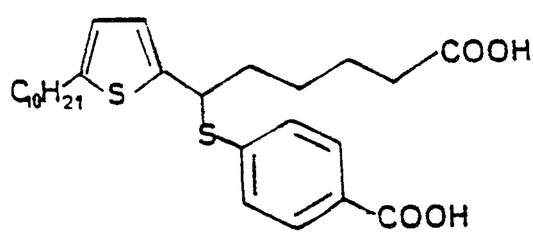

The corresponding US patent No. 4,609,744 issued without the claims covering the benzoic acid group.

The EP '543 application, and the one discussed below, contain a simple statement that all these compounds are active as antagonists of $LTC_4$, $LTD_4$, $LTE_4$ and SRS-Aas detected by known methods, with reference to a patent describing compounds that are active on guinea-pig ileum.

EP 147217 to Merck claims compounds of a general formula (C) which include the natural form of R in the description.

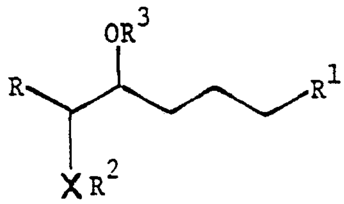

R is described as a variety of groups including carboxyl and esters thereof and $R^3$ may be H. X is defined as O, S, SO and $SO_2$ with $R^2$ being chosen from a variety of aromatic groups. $R^1$ is defined as COOR″, $CH_2OH$, CHO, tetrazole, hydroxymethylketone, CN, $CON(R^4)_2$, $CONHSO_2R^5$, a monocyclic or bicyclic heterocyclic ring containing an acidic hydroxyl group or $NHSO_2R^5$ or R″ can be hydrogen.

By judicious selection, a compound could contain the natural tail as R, $R^1$ = COOH and $R_3$ as H obtaining the structure

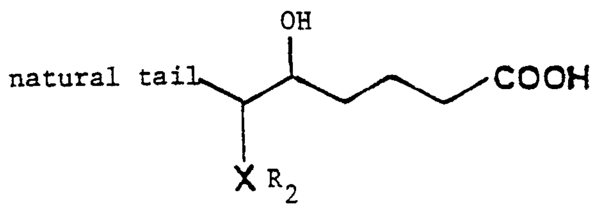

The nearest definition to a benzoic acid is for $R_2 = C_6H_4COCH_2OH$.

## Summary of the Invention

The invention provides leukotriene antagonists having the natural tail found in leukotrienes themselves with modifications in the peptide and head portion.

Broadly, leukotriene antagonists are claimed comprising compound I, esters, amides and physiologically acceptable salts thereof, where compound I is represented by the formula:

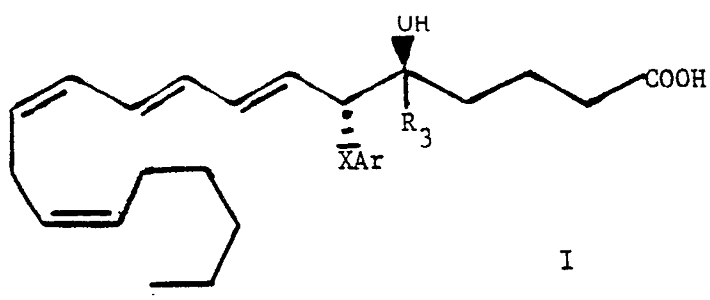

I

wherein $R_3$ is H or a lower alkyl group of 1 to 6 carbon atoms; and XAr is selected from

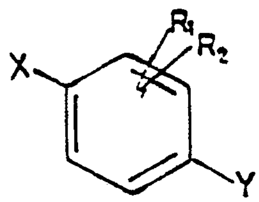

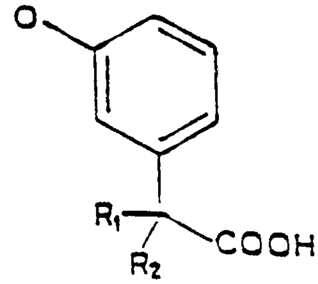

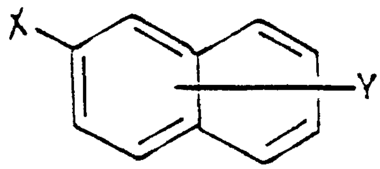

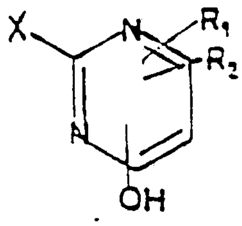

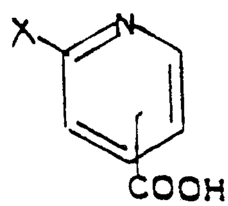

where X is selected from S,O, SO, $SO_2$ or $SCH_2$;
$R_1$ and $R_2$ are, same or different, H, lower alkyl, lower alkoxy, alkoxyalkyl, or $C_6H_4COOH$; and
Y is selected from H, OH, $CO_2H$, $CHN_4$, $CONH_2$, $CO_2R_1$, $OR_1$, $CH(CO_2H_2)_2$, $CR_1R_2CO_2H$ or $C_6H_4COOH$, with the proviso that when $R_3$ is hydrogen, XAr is not

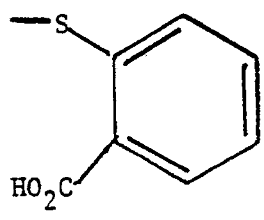

or

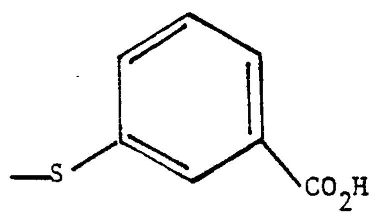

A preferred amide is also claimed, comprising compounds of formula II:

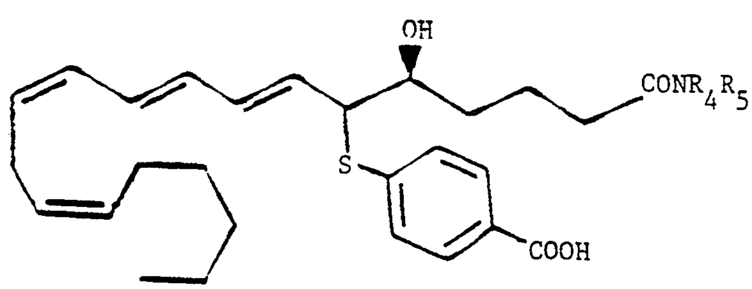

II

wherein $R_4$ and $R_5$, same or different, are each selected from H, alkyl or aryl groups, optionally substituted by one or more carboxyl or amino groups or $R_4$ and $R_5$ together with the nitrogen of the amide group from a heterocyclic ring.

## Detailed Description of the Invention

Interest in "Slow Reacting Substance of Anaphylaxis" (SRS-A) and other arachidonate metabolites has been heightened by the identification of the chemical identities that comprise SRS-A. SRS-A obtained from laboratory animals and from humans has been shown to consist of mixtures of leukotriene $C_4$ ($LTC_4$) and leukotriene $D_4$ ($LTD_4$).

$LTC_4$ and $LTD_4$ have been proposed as primary mediators in human asthma which are released from mast cells and basophils on antigenic challenge. $LTC_4$ and $LTD_4$ have been shown to be potent constrictors of human airway smooth muscle. SRS-A also produces permeability changes in skin tissues and may be involved in acute cutaneous allergic reactions. It has also been shown to depress ventricular contraction and to potentiate the cardiovascular effects of histamine.

In view of the potent effects of $LTC_4$ and $LTD_4$ on airway smooth muscle it is desirable to have available compounds which block the actions of these leukotrienes on smooth muscle by pharmacological antagonism. Such compounds are referred to as leukotriene antagonists. This invention describes compounds of structure (I) which possess this property. Some of these compounds also inhibit the biosynthesis of $LTC_4$.

Compounds (I) are prepared by reaction of the appropriate (thio)phenol, or thiol, with leukotriene $A_4$ methyl ester, ($R_3$ = H) or leukotriene $A_4$ methyl ester substituted at $C_5$ by a lower alkyl group:

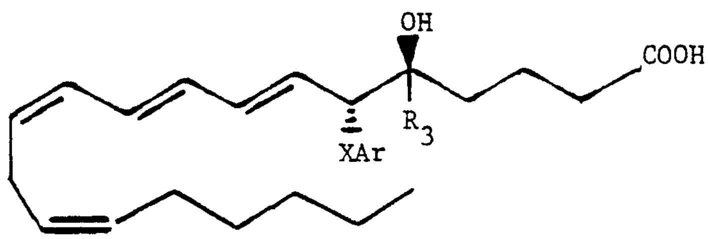

wherein X is chosen from -S-, -O-, -SO-, $-SO_2-$ or $-SCH_2-$. The $LTA_4Me$ ester can be prepared by the method disclosed in Example 1. The $LTA_4$-methyl ester substituted by a lower alkyl, preferably methyl group, can be prepared as shown in Examples 49-64.

The leukotriene antagonist activity of the compounds of this invention is measured by the ability of the compounds to inhibit the leukotriene induced contraction of guinea-pig ileum in vitro as described below.

Five (5) centimeter longitudinal strips of guinea-pig ileum (Male Dunkin-Hartley strain) were bathed in modified Tyrodes buffer, containing 3μM indomethacin, in jacketed 10 mL tissue baths at 37° C and continuously aerated with 95% $O_2$/5% $CO_2$. The tissues were connected via cotton sutures to isotonic transducers. The tissues were equilibrated for 60 minutes and 100μM histamine was added to determine the maximal response of the tissues. This was removed by washout to give a steady baseline. Then the test compound, or vehicle control, was added cumulatively in half-log units until a bath concentration of 10μM was reached.

After a further equilibration period of 15 minutes, a concentration response curve to the reference agonist ($LTD_4$ or $LTC_4$) was generated. All determinations were carried out in triplicate and observations normalised against the maximal leukotriene response. Least-squares linear regression analysis was then used to calculate the concentration of leukotriene needed to elicit 50% of the maximum response and defined as the $EC_{50}$.

The negative logarithm of the antagonist affinity constant ($pK_B$) is then given by the equation $pK_B = \text{Log}\,[10^{-5}/(DR-1)]$

where the dose ratio (DR) =

$$\frac{EC_{50}\ \text{(presence of test compound)}}{EC_{50}\ \text{(absence of test compound)}}$$

The values determined for the compounds disclosed in the examples are given in Table 1. All compounds were measured against $LTD_4$ as the reference agonists except the compound of Example 65

which were measured against $LTC_4$.

Table 1

| Compound of Example No. | MLS No. | $pK_B$ |
|---|---|---|
| 6 | 6210 | 7.0 |
| 7 | 6227 | 6.5 |
| 8 | 6220 | 5.9 |
| 9 | 6216 | 7.2 |
| 11 | 6222 | 7.5 |
| 10 | 6218 | 7.0 |
| 14 | 6228 | 6.0 |
| 15 | 7337 | 5.8 |
| 16 | 6238 | 6.4 |
| 18 | 6261 | 6.5 |
| 19 | 6262 | 6.3 |
| 17 | 6252 | 6.4 |
| 20 | 6257 | 6.1 |
| 21 | 6258 | 6.1 |
| 22 | 6256 | 6.6 |
| 23 | 6226 | 6.5 |
| 24 | 6243 | 5.5 |
| 25 | 6245 | 6.1 |
| 26 | 7330 | 6.4 |
| 27 | 6204 | 6.0 |
| 28 | 6271 | ND |

| Compound of Example No. | MLS No. | $pK_B$ |
|---|---|---|
| 29 | 6276 | ND |
| 30 | 6281 | ND |
| 31 | 6282 | ND |
| 65 | 7356 | 6.4* |

ND=not determined

*against $LTC_4$

The compounds of formula I all have the natural tail as found in leukotrienes themselves. Modification of the peptide and or head portion has produced useful antagonist activity.

The compounds have the general formula shown below:

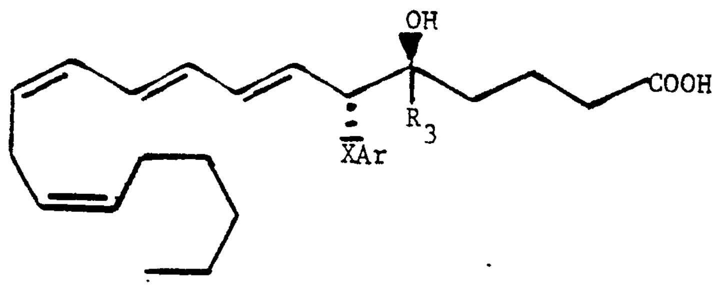

I

wherein $R_3$ is H or a lower alkyl group of 1 to 6 carbon atoms; and XAr is selected from

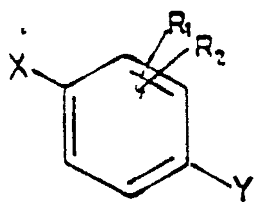

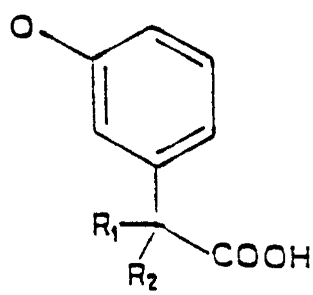

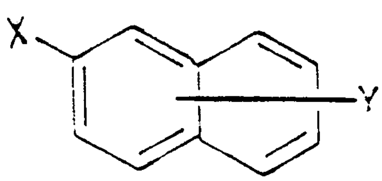

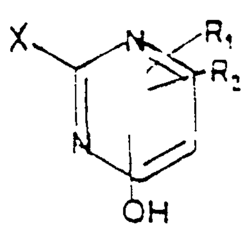

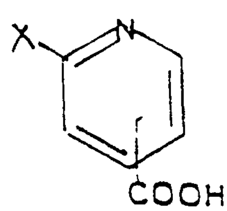

where X is selected from S,O, SO, $SO_2$ or $SCH_2$
$R_1$ and $R_2$ are, same or different, H, lower alkyl, lower alkoxy, alkoxyalkyl, or $C_6H_4COOH$; and
Y is selected from H, OH, $CO_2H$, $CHN_4$, $CONH_2$, $CO_2R_1$, $OR_1$, $CH(CO_2H_2)_2$, $CR_1R_2CO_2H$ or $C_6H_4COOH$.

Because compounds wherein $R_3$ is hydrogen and XAr is

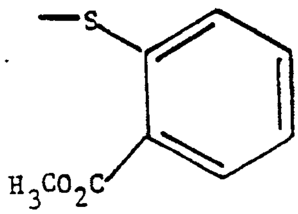

or

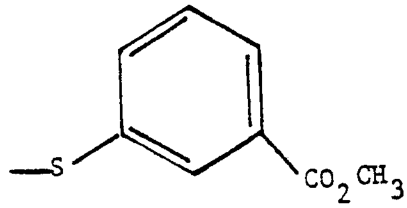

have been found to have agonist activity, those compounds have been specifically excluded from the claims by proviso.

Compounds include those wherein Y is hydrogen, $R_1$ is $C_6H_4COOH$ and $R_2$ is hydrogen, lower alkyl, lower alkoxy or an alkoxyalkyl group.

Lower alkyl is defined herein to include an unsubstituted or substituted chain of one to six carbon atoms.

Lower alkoxy is defined herein to include $C_1$-$C_6$ alkooxy and branched carbon chains such as isopropoxy.

Alkoxyalkyl groups include $C_1$-$C_6$ and branched chains alkoxymethyl or alkoxyethyl.

A second group of compounds is composed of compounds of formula I wherein $R_3$ is hydrogen and XAr is selected from

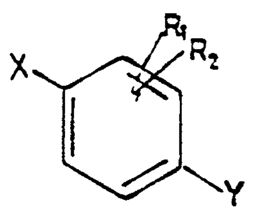

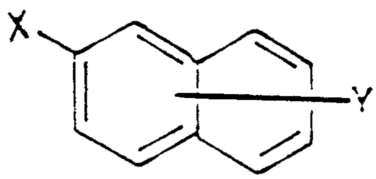

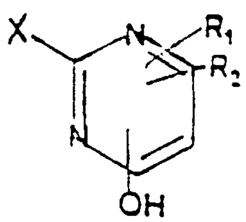

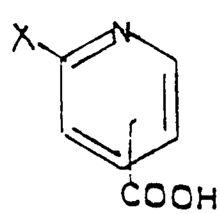

where $R_1$ and $R_2$, same or different, are H, lower alkyl, or alkoxyalkyl; X is selected from -O-, -S- or -$SCH_2$-; and
Y is selected from OH, $CO_2H$, $CHN_4$, $COHN_2$, $CO_2R_1$, $OR_1$, $CH(CO_2H_2)_2$ or $CR_1R_2CO_2H$.

A third preferred class of compounds is composed of compounds wherein $R_3$ is hydrogen and XAr is selected from

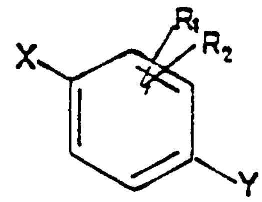

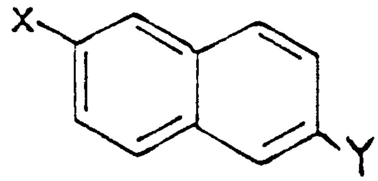

wherein $R_1$ and $R_2$, are same or different, H, lower alkyl or alkoxyalkyl; and Y is selected from OH, $CO_2H$, $CHN_4$, $COHN_2$, $CO_2R_1$, $OR_1$, $CH(CO_2H_2)_2$ or $CR_1R_2CO_2H$.

Most preferred are compounds shown below

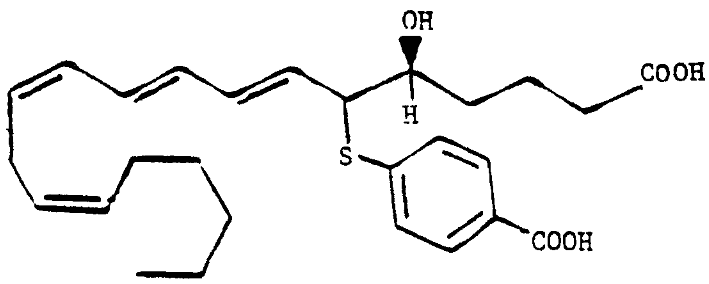

(MLS 6210)

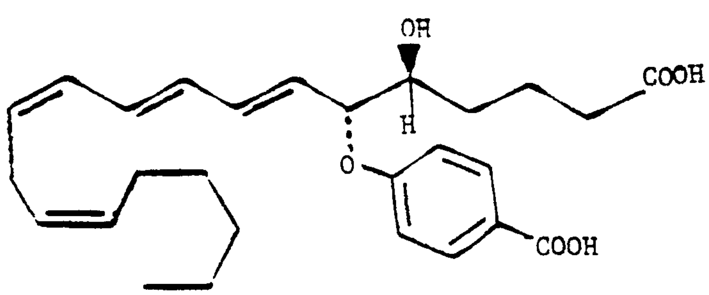

(MLS 6216)

wherein $R_3$ is hydrogen and XAr is chosen from

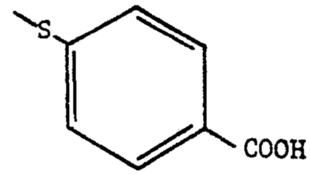

;

and

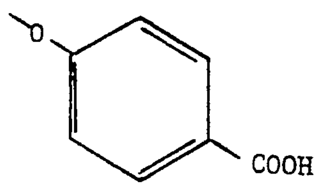

These compounds have been shown to be particularly good antagonists as shown in TABLE 1. The preparation of these compounds is detailed in Examples 6 and 9.

The leukotriene antagonists shown in formula I can also be used as esters, amides and physiologically active salts thereof. Such esters include methyl, ethyl, isopropl and amyl. Esterification may provide a protective function on administration so that upon metabolism to the carboxylic acid, the active component, is released. Physiologically active salts such as sodium or ammonium may provide solubility advantages in formulations such as nasal sprays.

A preferred group of amides, comprising compound II shown below

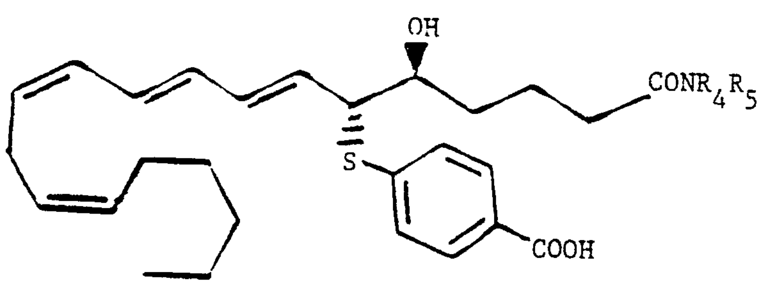

II

wherein $R_4$ and $R_5$, same or different are selected from H, a lower alkyl or aryl group, optionally substituted by one or more carboxyl or amino groups, or $R_4$ and $R_5$ together with the nitrogen form a heterocyclic ring.

The alkyl and aryl groups are preferrably unsubstituted, most preferably $-CH_3$, phenyl or naphthyl. When $R_4$ and $R_5$ together with nitrogen form a heterocyclic ring, it is preferably a 5- or 6-membered ring.

Leukotriene antagonists can be administered in a tablet, by intraveneous infusion or nasal spray to break up or delay onset of asthma-like symptoms.

The following examples are provided as a means of illustrating the present invention and are not to be construed as limiting the invention which is solely defined by the claims.

## EXAMPLES

Example 1 : Preparation of $LTA_4$-Me

3(Z)-Nonenyltriphenylphosphonium tosylate

1-0-p-Toluenesulphonyl-3(Z)-nonene (19.3g, 65mmol) and recrystallized triphenylphosphine (17.0g, 65mmol, equivalent) were heated under gentle reflux at 90° C in acetonitrile ($25cm^3$) for 48 hrs. The solvent was removed under reduced pressure and the product recrystallized from dichloromethane/diethyl ether ($100cm^3/150cm^3$) to give 3(Z)-nonenyltriphenylphosphonium tosylate (24.0g, 66% yield) as white needles m.p. 77-80° C.

The phosphonium tosylate (6.0g, 10.7mmol) was dissolved in $100cm^3$ dry tetrahydrofuran and cooled to -20° C under argon. n-Butyllithium solution (8.93mmol) in hexane was added dropwise and stirred for 5 minutes at -20° C before cooling to -78° C. After a further 10 minutes hexamethylphosphorous triamide ($18.6cm^3$, 12 equivalents) in tetrahydrofuran ($18.6cm^3$) was slowly added and stirred for 15 minutes before addition of the aldehyde methyl-11-oxo-5(S), 6(S)-oxidoundeca-7(E),9(E)-dienoate (1.0g, 4.46mmol) in tetrahydrofuran ($2cm^3$). The reaction mixture was stirred at -78° C for half an hour and then quenched with $1cm^3$ methanol, allowing the reaction temperature to slowly rise to room temperature.

The product was partitioned between diethyl ether and water, washed well with further water and brine, dried over magnesium sulphate and concentrated. The crude product was purified by rapid column chromatography on silica (previously deactivated by standing for 3 hours in the eluent); eluting with hexane/diethyl ether/triethylamine (88:10:2, $200cm^3$) under argon pressure. The eluent, upon concentration, yielded the desired $LTA_4$-Me (1.36g, 92% yield).
λmax (cyclohexane): 271, 280.3, 292.1nm

Example 2 : Preparation of Intermediates

Methyl 4-mercaptobenzoate

a) 4-mercaptobenzoic acid

4-Aminobenzoic acid (13.7g), sodium nitrite (6.9g) and sodium hydroxide (4.4g) were dissolved in water (80mL); then slowly added to 4M hydrochloric acid (107mL) at -5° C. The mixture was stirred for 1 hour; then a solution of ethyl potassium xanthate (4.8g) was added and the mixture heated to 70° during addition and for 1 hour. This was acidified with hydrochloric acid and cooled to give the disulphide as a beige precipitate which was collected by filtration.

This was refluxed for 24 hours with powdered zinc (25g) in acetic acid (200mL) then filtered and treated with concentrated hydrochloric acid (400mL). The resulting precipitate was thoroughly washed with water to leave a beige powder m.p. 219-221° C.

b) Methyl 4-mercaptobenzoate

The 4-mercaptobenzoic acid prepared above was dissolved in methanol (200mL) and refluxed for 4 hours with concentrated sulphuric acid (20mL). The solution was partially concentrated at reduced pressure and poured into water. The mixture was extracted with ether (2 x 120mL). The ether extracts were then washed with saturated sodium bicarbonate and brine, dried and concentrated to give cream crystals of methyl 4-mercaptobenzoate.
δ($60MH_z$,$CDCl_3$) 3.8 S 4H $CH_3$ and SH; 7.1 - 7.85 m 4H ArH.

Example 3 :

4-cyanothiophenol

a) 4 cyanophenyl-O-dimethylthiocarbamate

4-cyanophenol (25.6g) was dissolved in dimethylformamide and diazobicyclooctane (36.5g) was added followed by dimethylthiocarbamoylchloride (33.5g). After 5 hours, this was poured into water and the resulting precipitate collected and washed with water. This was recrystallised from chloroform-light petroleum to give cream needles m.p. 116-118 °C (35.7g).

b) 4-cyanophenyl-S-dimethylthiocarbamate

The product of step a (35.7g) was heated to 193 °C for 1 hour. After cooling it was recrystallised from ethanol to give (32.3g) mp 73-85 °C.

c) The product of step b (32.2g) was refluxed for 2 hours with sodium hydroxide (9.4g) in aqueous ethanol then acidified with hydrochloric acid and extracted with ether. The ether extracts were washed and dried to give the thiol as a colourless oil (21g).

Example 4 :

5(4-mercaptophenyl)-1(H)-tetrazole

4-cyanothiophenol (1g), ammonium chloride (1.6g) and sodium azide (1.9g) were heated for 2 hours at 150 °C in dimethylformamide (10mL), followed by 16 hours at 80 °C. The cooled solution was added to aqueous sodium bicarbonate and washed with chloroform. The aqueous phase was acidified with sulphuric acid giving the tetrazole as a white precipitate, m.p. 265-266 °C (0.73g).

The following leukotriene analogues were obtained by reaction of leukotriene $A_4$ methyl ester with the indicated (thio)phenol for the designated times following the procedure of Example 6. The thiophenols were obtained by following the method of Example 2 or Example 3 if not commercially available.

Example 5 :

Ethyl 1,1′-biphenyl-4′-mercaptomethyl-4-carboxylate

a) Ethyl 1,1′-biphenyl-4′-methyl-4-carboxylate

4-bromotoluene (1.71g) was dissolved in dry tetrahydrofuran (15mL) and cooled to -78 °C and 1.9M t-butyllithium solution (10.5mL) was added. After 15 minutes the solution was added to a suspension of zinc chloride (1.36g) in tetrahydrofuran and stirred 60 min at room temperature.

1M di-isobutylaluminiumhydride (0.53g) was added to palladium (II) bistriphenylphosphine dichloride (0.53g) in tetrahydrofuran (20mL) followed by ethyl 4-iodobenzoate (2.07g). After a few minutes, the zinc reagent was added and stirred for 60 minutes, then poured into 2M hydrochloric acid and extracted with ether. The extracts were washed with brine, dried and concentrated to give a red solid (2.47g). Chromatography on silica gel (100g) with 20% ether in hexane, gave an orange solid which recrystallised from aqueous ethanol as fine orange needles mp 71-72 °C (1.17g).

b) Ethyl 1,1′-biphenyl-4′bromomethyl-4-carboxylate

The product of step (a) (0.96g) and N-bromosuccinimide (0.68g) were refluxed overnight in carbon tetrachloride (25mL) in the presence of benzoylperoxide (30mg). The cooled solution was washed with sodium metabisulphite and brine, dried and concentrated to give a yellow solid (1.42g). Recrystallisation from aqueous ethanol gave the monobromide as fine cream needles, mp 90-91 °C (0.82g).

c) Ethyl 4′-(S-acetylthiomethyl)-1,1′-biphenyl-4-carboxylate

The product of step (b) (0.48g), potassium thioacetate (0.18g) and sodium iodide (50mg) were refluxed in acetone (50mL) for 2½ hours. The solvent was removed at reduced pressure and the residue partitioned between ethyl acetate and brine. The organic layer was taken to dryness and filtered through a pad of Florisil with 10% ether-hexane to give a cream solid (0.45g). Recrystallisation from hexane gave fine white needles, mp 74-75 °C (0.61g).

d) The product of step (c) (0.13g) was dissolved in methanol (5mL) and stirred for 60 minutes with sodium methoxide (30mg), then stirred a further 15 minutes after addition of ammonium chloride (30mg). The solvent was removed at reduced pressure and the residue partitioned between dichloromethane and brine. The organic phase was dried and concentrated to give a white powder, mp 79-80 °C (0.10g).

Nmr details for these compounds and the positional isomers synthesised in the same way are in TABLE 2.

TABLE 2

| R = | H | Br |
|---|---|---|
| 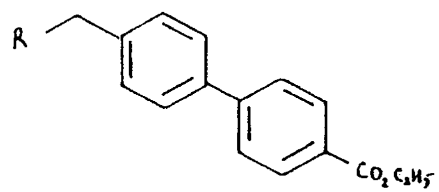 | 1.38 3Ht J=7Hz, 2.36 3Hs 4.34 2Hq J=7Hz, 7.16 2Hd J=7Hz, 7.45 2H d J=7Hz, 7.53 2H d J=8Hz, 8.01 2H d J=8Hz<br><br>Orange needles<br>mp 71-72°C (aq ethanol) | 1.32 3Ht J=7Hz, 4.26 2Hs, 4.35 2H q J=7Hz, 7.10 4H s, 7.16 2H d J=8Hz, 7.70 2H d J=8Hz<br><br>Cream needles<br>mp 90-91°C (aq ethanol) |
| 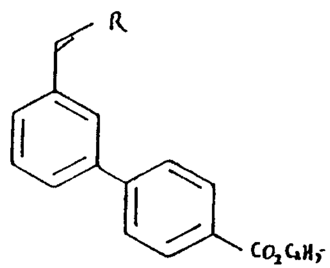 | 1.38 3H t J=7Hz, 2.38 3H s, 4.38 2H q, 7.0-7.3 2H m, 7.4 3H m, 7.70 2H d J=8Hz, 8.10 2H d J=8Hz.<br><br>Orange oil | 1.39 3H t J=7Hz, 4.33 2H q J=7Hz, 4.48 2H s, 7.2-7.6 6H m, 8.1 2H d J=8Hz<br><br>White needles<br>mp 87-88°C (ethanol) |
| 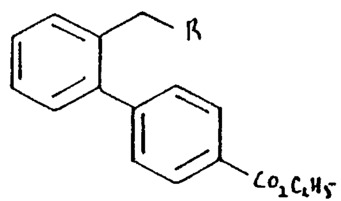 | 1.39 3H t, J=7Hz, 2.22 3H s, 4.35 2H q J=7Hz, 7.18 4H s, 7.30 2H d J=8Hz, 8.04 2H d, J=8Hz.<br><br>Yellow oil | 1.37 3H t, J=7Hz, 4.32 2H q J=7Hz, 4.27 2H s, 7.10 1H s, 7.20 1H d, J=2Hz, 7.25-7.4 2H m, 7.40 2H d, J=8Hz, 8.06 2H d J=8Hz.<br><br>White prisms<br>mp 75.5-76.5°C (ethanol) |

TABLE 2 Continued

| | $SCOCH_3$ | SH |
|---|---|---|
| 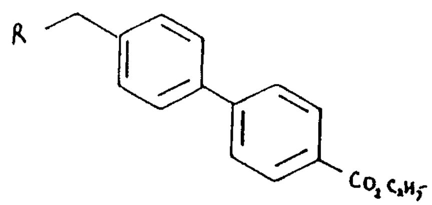 | 1.36 3H t, J=7Hz, 2.32 3H s, 4.09 2H s, 4.34 2H q, J=7Hz, 7.2-7.6 4H m, 7.54 2H d, J=8Hz, 8.03 2H J=8Hz<br><br>White needles<br>mp 74-75°C (hexane) | 1.38 3H t, J=7Hz, 1.75 1H t, J=8Hz, 3.72 2H d, J=8Hz, 4.38 2H q, J=7Hz, 7.2-7.7 6H m, 8.06 2H d J=8Hz.<br><br>λ max 281.4nM (MeOH)<br>White powder<br>mp 79-80°C |
| 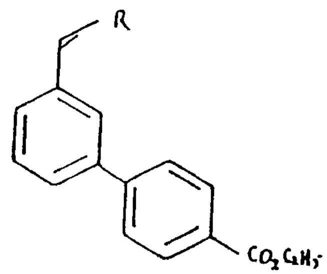 | 1.36 3H t, J=7Hz, 2.28 3H s, 4.10 2H s, 4.35 2H q, J=7Hz, 7.0-7.5 4H m, 7.55 2H d, J=8Hz, 8.05 2H d, J=8Hz.<br><br>Colourless oil | 1.35 3H t, J=7Hz, 1.76 1H t, J=7Hz, 3.66 2H d, J=7Hz, 4.34 2H q J=7Hz, 7.1-7.5 4H m, 7.54 2H d, J=8Hz, 8.03 2H d J=8Hz.<br><br>λ max 272,3nM (MeOH)<br>White powder<br>mp 40-42°C |
| 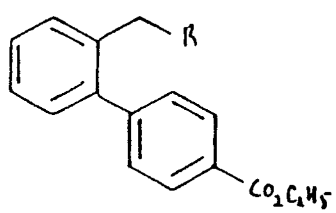 | 1.36 3H t, J=7Hz, 2.22 2H s, 4.02 2H s, 4.32 2H q J=7Hz, 7.0-7.4 4H m, 7.36 2H d J=8Hz, 8.08 2H d J=8Hz.<br><br>Colourless oil | 1.49 3H t J=7Hz, 1.64 1H t J=7Hz, 3.58 2H d J=7Hz, 4.35 2H q J=7Hz, 7.1-7.4 4H m, 7.38 2H d J=8Hz, 8.05 2H d, J=8Hz<br><br>λ max 255.6nM (MeOH)<br>Colourless oil |

TABLE 2 Continued

| R = | H | Br |
| --- | --- | --- |
| 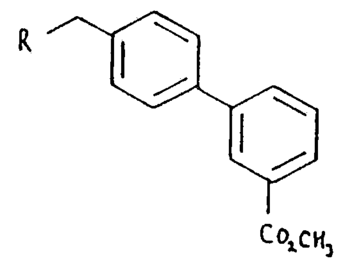 | 2.32 3H s, 3.87 3H s, 7.1-7.6 5H m, 7.70 1H dt J=7Hz, 2Hz 7.95 1H dt J=7Hz, 2Hz, 8.23 1H t, J=2Hz.<br><br>Pale yellow<br>mp 54-57°C | 3.79 3H s, 4.38 2H s, 7.15-7.45 5H m, 7.55 1H dt J=7, 2Hz, 2.95 1H dt J=7, 2Hz, 8.09 1H t, J=2Hz.<br><br>White needles<br>mp 90-91°C (cyclohexanone) |
| 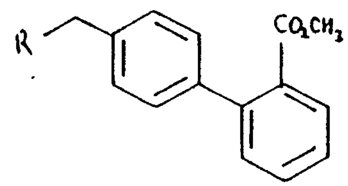 | 2.34 3H s, 3.59 3H s, 7.11 4H s, 7.2-7.4 3H m, 7.6-7.8 1H m.<br><br>Yellow oil | 3.60 3H s, 4.48 2H s, 7.2-7.6 7H m, 7.75-7.95 1H m.<br><br>Colourless wax |
| 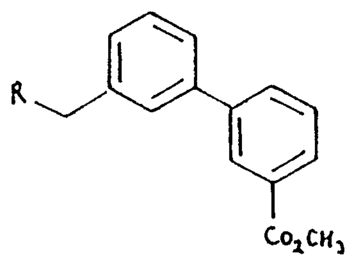 | 2.33 3H s, 3.84 3H s, 7.1-7.6 5H m, 7.66 1H dt, J=8, 1.5Hz, 7.91 1H dt J=7, 1.5Hz, 8.22 1H t J=1.5Hz.<br><br>Yellow oil | 4.00 3H s, 4.60 2H s, 7.4-8.0 6H m, 8.20 1H dt J=8, 1.5Hz, 8.42 1H t, J=1.5Hz.<br><br>Colourless oil |

TABLE 2 Continued

| | $SCOCH_3$ | SH |
|---|---|---|
| 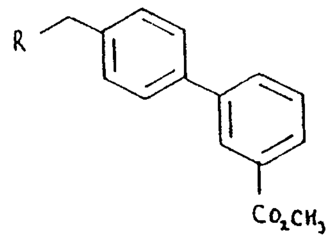 | 2.30 3H s, 3.82 s, 4.04 2H s, 7.0-7.4 5H m, 7.52 1H dt J=7, 2Hz, 7.78 dH dt J=7, 2H z, 8.00 1H t J=2Hz<br><br>White needles<br>mp 48-50°C (hexane) | 1.76 1H t J=7Jz, 3.72 2H d J=7Hz, 3.90 3H s, 7.3 1H dd J=6, 2.3Hz, 7.35-7.6 4H m, 7.75 1H dt, J=7, 1.7Hz, 7.86 1H dt J=7, 1.3Hz, 8.26 1H t J=1.7Hz<br><br>λ max 234.4 (MeOH)<br>Colourless oil |
| 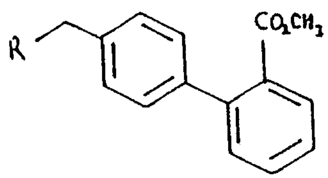 | 2.27 3H s, 3.52 3H s, 4.04 2H s, 7.0-7.4 7H m, 7.6-7.8 1H m.<br><br>Colourless oil | 1.75 1H t J=7Hz, 3.50 3H x, 3.60 2H m, 7.2-.75 7H m, 7.7-7.9 1H m<br><br>λ max < 220nM<br>Colourless oil |
| 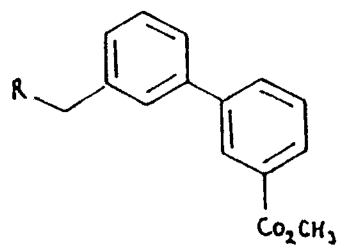 | 2.25 3H s, 3.78 3H s, 4.01 2H s, 7.0-7.3 5H m, 7.5 1H dt J=8, 1.5 Hz, 7.75 1H dt J=8, 1.5Hz, 7.98 1H t J=1.5Hz.<br><br>Colourless oil | 1.80 1H t J=7Hz, 3.60 2H m, 3.90 3H s, 7.2-7.6 5H m, 7.75 1H dt J=8, 1.5Hz, 8.02 1H dt J=8, 1.5Hz, 8.28 1H t J=1.5Hz.<br><br>λ max < 200nM<br>Colourless oil |

Example 6 :

5(S)-Hydroxy-6(R)-S-(4′-carboxyphenylthio)-7(E), 9(E),11(Z),14(Z)-eicosatetraenoic acid MLS 6210

Leukotriene $A_4$ methyl ester (0.3g) in methanol: triethylamine ($3cm^3$, 1:1) was stirred under argon at room temperature with 10 equivalents of methyl 4-mercaptobenzoate (1.5g) for 3 hours. Concentration to dryness and dissolution in 1mL methanol was followed by preparative thin layer chrmoatography (TLC) on Whatman PLKC18F reversed phase plates, 1000$\mu$m thickness. Further purification was performed using preparative high pressure liquid chromatography (HPLC) (Zorbax ODS 25cm x 21.1mm i.d., $CH_3CN$-$H_2O$ 80-20, $18cm^3$/min, UV 280nm) to yield 132mg (29%) of the diester ($t_r$ 20 min). Treatment with tetrahydrofuran (THF 2.0mL) and aqueous lithium hydroxide (0.126g, 2mL deoxygenated water) under argon at room temperature for 20 hours afforded the title compound (130mg, 28% yield).
$\lambda$max (methanol) 278.5nm
FAB MS M- 471

Example 7 :

5(S)-Hydroxy-6(R)-S-(4′carboxyphenylthio)-7(E),9(E), 11(E),14(Z)-eicosatetraenoic acid MLS 6227

HPLC of the diester of Example 7 separated two components. The later eluting of these ($t_r$ 23.3 min) was collected separately and hydrolyzed as in Example 6 to give the 11(E) isomer.
$\lambda$max 276.5nM

Example 8 :

5(S)-Hydroxy-6(R)-S-(4′methoxycarbonylphenylthio)-7(E),9(E),11(Z),14(Z)-eicosatetraenoic acid (MLS 6220)

The diester (15mg), prepared as described in Example 6, was dissolved in aqueous tetrahydrofuran 1mL with lithium hydroxide (10mg). After 30 minutes two peaks were evident by HPLC. The monoester and the diacid were separated by preparative HPLC as above collecting the later eluting peak (4.8mg).
$\lambda$max 278.4nM

Example 9 :

5(S)-Hydroxy-6(R)-O-(4′-carboxyphenoxy)-7(E),9(E), 11(Z),14(Z)-eicosatetraenoic acid MLS 6216

$LTA_4$ methyl ester (25mg) was stirred with methyl-p-hydroxybenzoate (100mg) in methanol: triethylamine (1mL, 1:1) for 24hours. The product was purified by preparative TLC followed by normal phase HPLC with hexane:ethyl acetate 70:30 to give the diester (8mg).
$\lambda$max ($CHCl_3$) 281nM
FAB M-H 483
This was saponified as described in Example 6 to give the title compound.
$\lambda$max (MeOH) 275.9nM
FAB M-H 455

Example 10 :

5(S)-Hydroxy-6(R)-S-(4′carboxymethylphenylthio)-7(E),9(E), 11(Z),14(Z)-eicosatetraenoic acid MLS 6218

Methyl 4-mercaptophenylacetate was prepared from 4-aminophenylacetic acid using the method of Example 2. This was reacted with $LTA_4$ methyl ester following the procedure of Example 6. The product was purified by normal-phase HPLC with hexane:EtOAc 70:30; then deprotected as in Example 6.

λmax 278.8nM
FAB ms(M+1) 486

TABLE 3

| Example No. | (Thio)phenol | Reaction Time Hours | λ max nM |
|---|---|---|---|
| 11 | 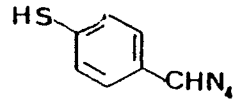 | 1 | 278.7 |
| 12 | 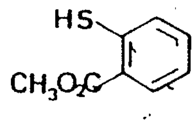 | 1 | 280.8 |
| 13 | 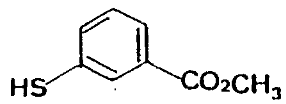 | 2.5 | 282.0 |
| 14 | 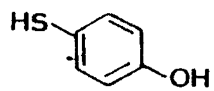 | 2 | 282.2 |
| 15 | 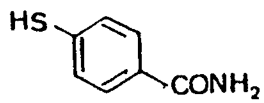 | ? | 278.0 |
| 16 | 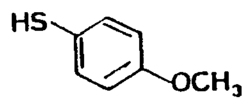 | 3 | 278.1 |

TABLE 3 Continued

| Example No. | (Thio)phenol | Reaction Time Hours | λ max nM |
|---|---|---|---|
| 17 | 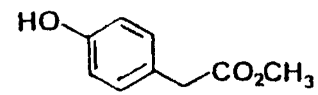 | 4 | 276.6 |
| 18 | 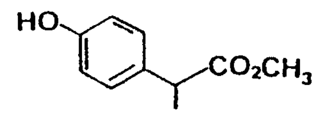 | 7 | 274.9 |
| 19 | 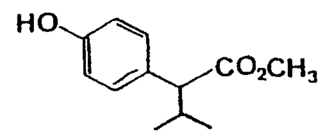 | 24 | 275.2 |
| 20 | 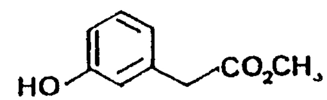 | 4.5 | 274.1 |
| 21 | 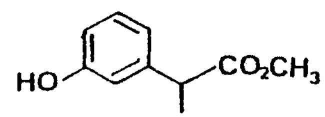 | 8 | 275.7 |
| 22 | 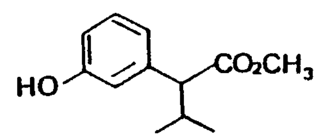 | 24 | 278.4 |

TABLE 3 Continued

| Example No. | (Thio)phenol | Reaction Time Hours | λ max nM |
|---|---|---|---|
| 23 | 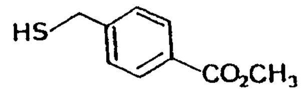 | 2 | 281.5 |
| 24 | 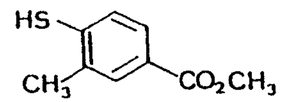 | 5 | 278.1 |
| 25 | 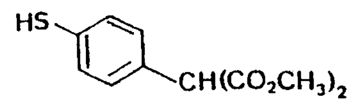 | 2 | 280.7 |
| 26 | 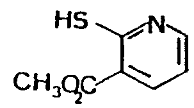 | ? | 281.0 |
| 27 | 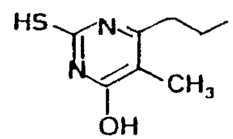 | 5 | 280.4 |
| 28 | 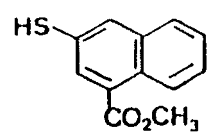 | 2 | 273.7 |

TABLE 3 Continued

| Example No. | (Thio)phenol | Reaction Time Hours | λ max nM |
|---|---|---|---|
| 29 | 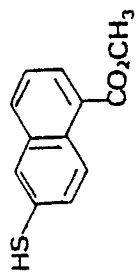 | 2 | 276.2 |
| 30 | 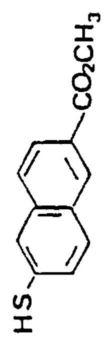 | 2 | 267.4 |
| 31 | 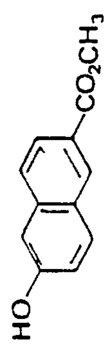 | 5 | 277.0 |

The leukotriene analogs of Examples 32 to 39 were prepared following the general procedure of Example 6. The thiols of examples 34 to 39 were prepared following the general procedure of Example 5.

TABLE 4

| Example No. | (Thio)phenol | Reaction Time Hours | λ max nM |
|---|---|---|---|
| 32 11(Z) | 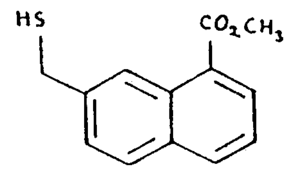 | 4.5 | 281.3 |
| 33 11(E) | | | 281.4 |
| 34 | 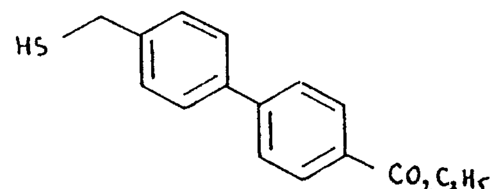 | 2 | 281.7 |
| 35 | 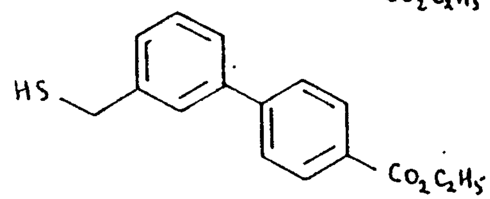 | 2 | 279.6 |
| 36 | 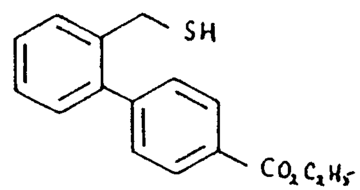 | 2.5 | 270, 280.7 |

TABLE 4 Continued

| Example No. | (Thio)phenol | Reaction Time Hours | λ max nM |
|---|---|---|---|
| 37 | 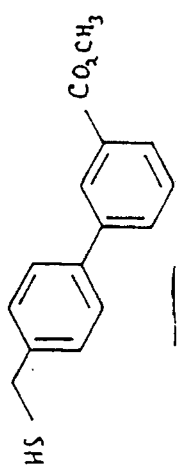 | 3 | 281.0 |
| 38 | 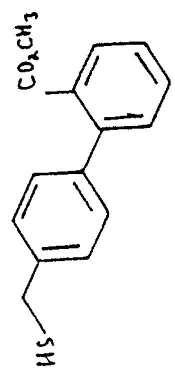 | 4.5 | 281.1 |
| 39 | 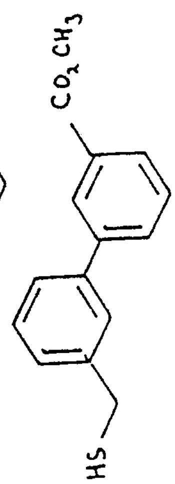 | 3 | 281.0 |

The thiols of examples 34 to 39 were prepared following the general procedure of Example 5.

Example 40 :

N-butyl 5(S)-hydroxy-6(R)-(4′-carboxyphenylthio)-7(E),9(E), 11(Z),14(Z)-eicosatetraenamide

The product of Example 8 (87mg) was dissolved in dimethylformamide (1mL) and diphenylphosphoryl azide (58mg), butylamine (25mg) and triethylamine (48mg) were added. The solution was stirred, under argon, at 0 °C for 3 hours. This was poured into water and extracted with ether (40mL). The ether solution

was washed with water (4 x 10mL), dried and concentrated to dryness then purified by preparative HPLC as described in Example 6.

This gave the amide (29mg).λmax (MeOH) 278.7nM $t_R$ 9.0 min (μ-Bondapak $C_{18}$ MeCN:$H_2O$ 80:20 1mL/min)

The ester was hydrolysed using the method of Example 6 to give the title compound (19.5mg) $t_R$ 5.3 min.

Examples 41 - 48 :

Reaction of the product of Example 8 with the indicated amine following the method of Example 40 gave the appropriate amide ester which was hydrolysed as described in Example 6.

TABLE 5

| Example No. | Amine | λ max nM | Reaction Time Hours | HPLC Retention Times Min | |
|---|---|---|---|---|---|
| | | | | Ester | Acid |
| 41 | Ethyl Glycinate Hydrochloride | 278.5 | 24 | $6.0^{b}$ | $11.5^{c}$ |
| 42 | Benzylamine | 278.2 | 3 | $8.1^{d}$ | 6.1 |
| 43 | Methyl 4-Aminobutyrate | 278.6 | 18 | $14.3^{e}$ | 3.7 |
| 44 | Methyl 4-Aminomethylbenzoate | 278.1 | 4 | 9.7 | 3.8 |
| 45 | Methyl α-amino-4-hydroxyphenylacetate Hydrochloride | 278.4 | 18 | 9.1 | $4.1^{d}$ |
| 46 | Methyl α-Aminophenylacetate Hydrochloride | 278.2 | 2 | $14.2^{d}$ | $4.4^{d}$ |
| 47 | Dimethyl Glutamate | 278.0 | 2 | $18.1^{gh}$ | $10.4^{ch}$ |
| 48 | Methyl α-Amino-4-Methoxycarbonylphenylacetate | 278.4 | 5 | $7.0^{b}$ | $7.3^{c}$ |

a) HPLC was carried out on a Lichrosorb $C_{18}$ RP column (25 x 0.4 cm) with acetonitrile:water:acetic acid at 1ml/min in the ratio 80:20:0.1 except where indicated.
b) 90:10:0, c) 40:60:0.1, d) 80:20:0, e) 75:25:0, f) 60:40:0.1, g) 70:30:0.1
c) 40:60:0.1,
d) 80:20:0,
e) 75:25:0,
f) 60:40:0.1,
g) 70:30:0.1
h) a μ-Bondapak $C_{18}$ column (30 x 0.39 cm) was used.

Example 49 :

Geranyl acetate

To 130 g geraniol and 200 mL pyridine at 10°C was added 200 mL acetic anhydride followed by 0.4 g 4-dimethylaminopyridine. After stirring for one hour, the mixture was poured onto crushed ice and diluted with 200 mL n-hexane. The organic layer was separated, washed with 10% hydrochloric acid, 10% aqueous sodium bicarbonate and dried over magnesium sulfate ($MgSO_4$). The extract was evaporated in-vacuo to give the title compound (98%) as a colorless oil. This material was used in the subsequent transformation as obtained.

Example 50 :

6-Acetoxy-4-methylhex-4E-enal

The title compound was prepared in 57% yield from geranyl acetate (see Example 49), using the ozonolysis-zinc-acetic acid reduction procedure, taught by E. J. Corey, K. Achiwa, J. A. Katzenellenbogen, J. Amer. Chem. Soc. , 91, 4318 (1969).
Analytical Data : NMR ($CDCl_3$)δ: 1.8 (3 H, bs), 2.1 (3 H, s), 2.4-2.8 (4 H, m), 4.6 (2 H, bd, J=2 Hz) 5.4 (1 H, bt, J=7 Hz), 9.9 (1 H, bt).

Example 51 :

6-Acetoxy-4-methylhex-4E-en-1-ol

To 30.6 g 6-acetoxy-4-methylhex-4E-enal (Example 50) in 180 mL dry methanol at 0° C, 6.84 g of sodium borohydride, was added portion-wise, with stirring over a 21 minute period. The reaction mixture was stirred for an additional 15 minute period and quenched with the addition of 20 mL water followed by the addition of 10.3 mL glacial acetic acid. The resultant solution was evaporated, in-vacuo , to a small volume and diluted with 150 mL n-hexane, 100 mL ether and 100 mL saturated aqueous brine. The organic layer was washed with 50 mL water and dried ($MgSO_4$). The aqueous layers were back extracted with 2 x 150 mL 2:1(v/v) n-hexane-ether. The combined extracts were evaporated in-vacuo to yield 25.8 g (83%) of the title compound as a pale yellow oil. This material was used in the following reaction as obtained.
Analytical data : NMR ($CDCl_3$)δ: 1.7 (3 H, s), 1.5-2.3 (4 H, m), 2.1 (3 H, s), 3.2 (1 H, bs), 3.6 (2 H, t, J=6 Hz), 4.6 (2 H, bd, J=7 Hz), 5.4 (1 H, bt, J=7 Hz).

Example 52 :

6-Acetoxy-4-methylhex-4E-enyl-p-toluene sulfonate

To a solution containing 62.4 g of 6-acetoxy-4-methylhex-4E-en-1-ol (Example 51) in 355 mL dry pyridine, at 5° C, 86.2 g of p-toluenesulfonyl chloride was added, over 8 minutes. The reaction mixture was stirred at 5° C for 15 minutes and at ambient temperature for 2.2 hours. The reaction mixture was poured onto 500 mL of an ice-water mixture and diluted with 300 mL chloroform. The organic layer was washed with 10% hydrochloric acid, water and dried over sodium sulfate ($Na_2SO_4$). The aqueous layers were back extracted with 2 X 200 mL 1:1 (v/v) n-hexane-ether. The combined extracts were evaporated in-vacuo to yield the title compound (118 g, 99%) as a red-orange oil. This material was used as obtained in the following transformation.
Analytical Data : NMR ($CDCl_3$)δ: 1.7 (3 H, bs), 1.6-2.2 (4 H, m), 2.1 (3 H, s), 2.5 (3 H, s), 4.1 (2 H, t, J=6 Hz), 4.6 (2 H, d, J=7 Hz), 5.3 (1 H, t, J=7 Hz), 7.4 (2 H, d, J=8 Hz), 7.8 (2 H, d, J=8 Hz); MS: 326 ($M^+$), 283 ($M^+$-$CH_3CO$); IR ($CHCl_3$): 3000, 1725, 1365 $cm^{-1}$.

Example 53 :

6-Acetoxy-1-bromo-4-methylhex-4E-ene

To 82.4 g dry lithium bromide in 3 L dry acetone at +5° C was added over 5 minutes, a solution containing 118 g of 6-Acetoxy-4-methylhex-4E-enyl-p-toluene sulfonate (Example 52) in 1 L dry acetone. The resultant solution was then stirred at ambient temperature for 19 hours and at 40° C for 0.5 hours. The reaction mixture was evaporated in-vacuo to a small volume and triturated with hot (50° C) n-hexane (1 L). The warm hexane solution was poured onto 250 mL silica gel contained in a sintered glass funnel. Elution with mixtures of hexane-ethyl acetate, combining like fractions and evaporation of solvents ( in-vacuo ) gave the title compound (74.1 g 87%), as yellow oil.
Analytical Data : MS (Cl-methane) m/e: 235 ($M^+$ +1), 233 ($M^+$ +1), 177 (base peak) (235 - Ac-$CH_3$), 175 (90.5%) (233 - Ac-$CH_3$).

Example 54 :

6-bromo-3-methylhex-2E-enol

To 22.1 g dry powdered potassium carbonate, was added with stirring a solution containing 25.0 g of 6-acetoxy-1 -bromo-4-methylhex-4E-ene (Example 53) in 200 mL methanol. After 35 minutes, the reaction mixture was added to a sintered glass funnel containing Celite® (pretreated with methanol). The filtrate was diluted with 200 mL of half saturated aqueous brine, 200 mL n-hexane and 14.7 g citric acid monohydrate. The organic layer was separated and washed with 100 mL saturated aqueous brine. The aqueous layers were back extracted with 1:1 (v/v) hexane-ethyl acetate and finally 4:1 (v/v) ethyl acetate-hexane. The combined organic extracts were dried ($MgSO_4$), and solvents evaporated in-vacuo to give 17.9 g of a pale orange oil. This material was purified by preparative high pressure chromatography (HPLC) using 75:25 (v/v) hexane-ethyl acetate as eluant. In this manner, the title compound was obtained (17.1 g, 83%) as a yellow oil.
Analytical Data : NMR ($CDCl_3$)$\delta$: 1.6 (3 H, s), 1.8-2.3 (4 H, m), 2.4 (1 H, bs), 3.4 (2 H, t, J=6 Hz), 4.1 (2 H, d, J=7 Hz), 5.4 (1 H, bt, J=7 Hz).

Example 55 :

6-bromo-3-methyl-1-trimethylsilyloxyhex-2E-ene

To a solution containing 2.0 g 6-bromo-3-methylhex-2E-en-1-ol (Example 54) in 20 mL dry methylene chloride at 0° C was added 2.0 mL triethylamine and 12.6 mg 4-dimethylaminopyridine. To this solution was added, dropwise, via syringe, 1.6 mL of trimethylsilyl chloride. The mixture was stirred at 0° C for 5 minutes and at ambient temperature for 1.0 hour. The reaction mixture was poured onto a mixture of ice-water and the organic layer washed with saturated aqueous brine and dried ($Na_2SO_4$). The aqueous layers were back extracted with 2 x 50 mL of ether. The combined dried extracts were evaporated in-vacuo to yield the title compound (2.6 g, 96%) as a pale yellow oil.
Analytical Data : NMR ($CDCl_3$)$\delta$: 0.5 (9 H, s), 1.6 (3 H, s), 1.7-2.3 (4 H, m), 3.4 (2 H, t, J=6 Hz), 4.1 (2H, d, J=7 Hz), 5.4 (1 H, bt, J=7 Hz).

Example 56 :

3-Methylocta-2,7E-dien-1-ol

To 10.54 g copper (I) cyanide in 155 mL dry tetrahydrofuran (THF) was added, at -20° C, 1.9 M vinyl lithium (124 mL) in THF. The black solution was stirred at -20° C to -15° C for 1.0 hr. To this solution was added a solution containing 12.5 g of 6-bromo-3-methyl-1-trimethylsilyloxyhex-2E-ene (Example 55) in 50 mL THF. The black solution was stirred at -20° C to -10° C for 3.1 hours and at 0° C for 2.5 hours. The reaction solution was poured into a 5° C stirred mixture containing 200 mL saturated aqueous ammonium chloride and 20 mL ammonium hydroxide. The blue-black mixture was diluted with 300 mL n-hexane and the layers separated. The organic layer was washed with a solution containing saturated aqueous amminium chloride (150 mL) and 15 mL ammonium hydroxide. The aqueous layers were back extracted with 200 mL of 2:1 (v/v) hexane-ethyl acetate and the combined organic extracts dried ($MgSO_4$) and evaporated. The resultant yellow oil was diluted with 50 mL ethanol and 10 mL 2% sulfuric acid and stirred at ambient temperature for 1.0 hour. The reaction mixture was evaporated in-vacuo to a small volume and diluted with ethyl acetate and saturated aqueous brine. The organic layer was dried ($MgSO_4$) and evaporated to yield an orange oil. This material was vacuum (3.0 torr) distilled (54° C - 57° C) to give the title compound (6.0 g, 91%) as a colorless oil.
Analytical Data : NMR ($CDCl_3$)$\delta$: 1.6 ( 3 H, s), 1.4-2.2 (7 H, m), 4.1 (2 H, d, J=7 Hz), 5.4 (1 H, bt, J=7 Hz), 5.0 (2 H, AB of an ABX pattern) 5.75 (1 H, X of an ABX pattern); CMR ($CDCl_3$) $\delta$: 138.3, 138.0, 123.6, 114.1, 58.5, 38.6, 33.0, 26.6, 15.7; IR ($CHCl_3$): 3600 (s), 3550-3225 (b), 2930, 1640, 1390, 1220, 990, 915 $cm^{-1}$, MS, m/e: 125 ($M^+$ -$CH_3$), 123 ($M^+$ - OH), 122 ($M^+$ - $H_2O$), 71 (base peak) ($C_4H_2O$).

Example 57 :

3-Methyl-2S,3S-epoxy-7-octenol

Epoxidation of 3-methylocta-2,7E-dien-1-ol (9.1 g) was done in accordance with T. Katsuki and K. B. Sharpless, J. Amer. Chem. Soc. , 102 , 5976, (1980) and J. G. Hill and K. B. Sharpless, "Organic Synthesis," volume 63, pp 66-78, J. Wiley & Sons, Inc. (1984) using titanium isopropoxide (0.051 mole), 14.3 g L(+)-diisopropyl tartrate, and 2.71 M anhydrous t-butylhydroperoxide (0.2 mole in toluene). By this procedure the title compound was obtained as a colorless oil (84%).
Analytical Data : $[\alpha]_D$ -7.22° (c 1.33, $CHCl_3$); NMR ($CDCl_3$)δ: 1.25 (3 H, s), 1.3-1.6 (4 H, m), 2.05 (2 H, m), 1.6 (OH, bt), 2.95 ($H_X$ of an ABX pattern), 3.75 ($CH_2OH$, AB of an ABX system), 5.0 (2 H, AB of an ABX system), 5.75 ($H_X$ of an ABX system).

Example 58 :

1-Acetoxy-3-methyl-2S,3S-epoxy-7-octene

To 8.5 g of the epoxyoctenol (Example 57) and 20 mL pyridine at 10°C was added 20 mL acetic anhydride followed by 0.04 g 4-dimethylaminopyridine. After stirring for one hour, the mixture was poured onto crushed ice and diluted with 20 mL n-hexane. The organic layer was separated, washed with 10% hydrochloric acid, 10% aqueous sodium bicarbonate and dried over magnesium sulfate ($MgSO_4$). The extract was evaporated in-vacuo to give the title compound (99%) as a pale orange oil.
Analytical Data : NMR ($CDCL_3$)δ: 1.25 (3 H, s), 2.05 (3 H, S), 2.95 ($H_X$ of an ABX pattern), 4.2 ($H_AH_B$ of an ABX pattern), 5.0 ($H_AH_B$ of an ABX pattern), 5.75 ($H_X$ of an ABX pattern).

Example 59 :

7-Acetoxy-5-methyl-5S,6S-epoxyheptanal

Ozonolysis was conducted in a similar manner to that described in S. L. Schreiber, R. E. Clans and J. Reagan, Tetrahedron Lett., 3867 (1982). To a stirred solution of 1-acetoxy-3-methyl-2S,3S-epoxy--7-octene (10.85 g) (Example 58) in 300 mL 5:1 (v/v) methylene chloride-methanol and 1.5 g sodium bicarbonate at -70°C was introduced a stream of ozone (Welsbach Ozonator). The reaction was monitored by TLC and when starting material was consumed, the mixture was poured into a flask containing benzene (55 mL). The solvents were evaporated in-vacuo to give an orange oil. The oil was cooled to 5°C and 9.6 mL triethylamine and 5.2 mL acetic anhydride were sequentially added. The reaction mixture was stirred at 5°C for 5 minutes at ambient temperature for 20 minutes, then diluted with chloroform and ice water. The organic layer was washed with 2% sulfuric acid, 10% sodium bicarbonate and saturated aqueous brine. The aqueous layers were back extracted with ethyl acetate and the combined dried ($Na_2SO_4$) extracts evaporated in-vacuo to give a pale orange oil. The oil was purified by HPLC using 65:35 (v/v) hexane-ethyl acetate eluant. Combining of like fractions and evaporation of solvents in-vacuo gave the title compound as a colorless oil (61%). The corresponding methylester ( cf Example 60) was obtained as a minor product in 27% yield.
Analytical Data (11c) : NMR ($CDCl_3$)δ: 1.3 (3 H, s), 1.3-2.0 (4 H, m), 2.1 (3 H, s), 2.5 (2 H, t), 3.0 ($H_X$ of an ABX pattern), 4.2 ($H_AH_B$ of an ABX pattern), 9.8 (1 H, t).

Example 60 :

Methyl 7-Acetoxy-5-methyl-5S,6S-epoxyheptanoate

Adapting the ozonolysis procedure of P. Sundaraman, E. C. Walker and C. Djerassi, Tetrahedron Lett.,

1627 (1978) but substituting dry methylene chloride for methanol as solvent and using 2.1 mole equivalents of lithium methoxide in methanol (1.8 M), the title compound was obtained from 6.65 g 7-acetoxy-5-methyl = 5S,6S-epoxyheptanal (Example 59) as a colorless oil after purification by silica gel chromatography 7:3 (v/v) hexane-ethyl acetate as eluant.

Analytical Data : NMR ($CDCl_3$)δ: 1.3 (3 H, s), 1.4-2.5 (6 H, m), 2.1 (3 H, s), 3.0 ($H_X$ of an ABX pattern) 3.65 (3 H, s), 4.2 ($H_AH_B$ of an ABX pattern); IR ($CHCl_3$): 3000, 2950, 1735, 1380, 1220, 1040 $cm^{-1}$.

Example 61 :

Methyl 7-Hydroxy-5-methyl-5S,6S-epoxyheptanoate

To 4.37 M sodium methoxide in methanol (0.05 mole equivalent) was added 54 mg methyl 7-acetoxy-5-methyl-5S,6S-epoxyheptanoate (Example 60). After 15 minutes, the mixture was filtered through a pad of Celite® (pretreated with methanol). The filtrate was diluted with half saturated brine and 1:1 (v/v) hexane-ethyl acetate. After drying the organic layer ($Na_2SO_4$) and evaporation of solvents in-vacuo the title compound was obtained (64%) as a colorless oil.

Analytical Data : $[\alpha]_D$ -6.3° (c 1.68, $CHCl_3$); NMR ($C_6D_6$)δ: 1.0 (3 H, s), 1.1-1.7 (5 H, m), 2.0 (2 H, t), 2.7 (1 H, t), 3.3 (3 H, s), 3.4 (2 H, d); IR ($CHCl_3$): 3600 (s), 3550-3300 (b), 3,000, 2950, 1730, 1440, 1220, 1030 $cm^{-1}$; MS (CI - methane) m/e: 189 ($M^+$ +1), 171 (189 - $H_2O$), 139 (base peak) ($C_8H_{11}O_2$).

Example 62 :

Methyl 7-Oxo-5-methyl-5S,6R-epoxyheptanoate

Collins reagent was prepared from the addition of 343 mg chromium trioxide in 0.55 mL dry pyridine and 8 mL dry methylene chloride at 0° C. The mixture was stirred at ambient temperature for 0.5 hours. Dry celite (1.1 g) was added to the deep burgundy solution. To this, Collins reagent at 0° C was added to a solution of 93 mg methyl 7-hydroxy-5-methyl-5S,6S-epoxyheptanoate (Example 61) in 2 mL dry methylene chloride. The reaction mixture was stirred at ambient temperature for 0.6 hours and poured onto a sintered glass funnel containing 25 mL silica gel pretreated with 1:1 (v/v) hexane-ether. The pad was eluted with 2 x 15 mL portions of 1:1 (v/v) hexane-ether. Combination of like fractions and evaporation of solvents in-vacuo gave the title compound (81%) as a colorless oil.

Analytical Data : NMR ($CDCl_3$)δ: 1.4 (3 H, s), 1.6-1.8 (4 H, m), 2.3 (2 H, t), 3.2 (1 H, d, J = 4.9 Hz), 3.7 (3H, s), 9.5 (1 H, d, J = 4.9 Hz).

Example 63 :

Methyl 11-oxo-5-methyl-5S,6S-epoxyundeca-7E,9E-dienoate .

The methods of I. Ernst, et al., Tetrahedron Lett., 167 (1982) and N. Cohen, et al., J. Amer. Chem. Soc., 105 , 3661 (1983) were used to obtain the title compound from 1.18 g methyl 7-oxo-5-methyl-5S,6R-epoxyheptanoate (Example 62). After purification by silica gel column chromatography (7:3:0.01 v/v/v hexane-ethyl acetate-triethylamine as eluant), this intermediate was used immediately in the subsequent reaction.

Example 64 :

Methyl 5-Methyl-5S,6S-epoxyeicosa-7E,9E,11Z,14Z-tetraenoate

To 198 mg of 3Z-nonenyltriphenylphosphonium tosylate (prepared as shown in Example 1) in 3.9 mL

dry tetrahydrofuran, under argon, at -40°, was added n-butyllithium (1.35M in n-hexane, 0.24 ml). After 40 minutes 0.60 mL dry hexamethylphosphoramide was added and the orange-yellow mixture cooled to -70° C. To this mixture was added a solution containing 10 mg of methyl 11-oxo-5-methyl-5S,6S-epoxyundeca-7E,9E-dienoate (Example 63) in 1.5 mL THF. The orange solution was stirred at -70° C for 30 minutes, 12 microliters dry methanol was added; the yellow solution stirred at 0° C for 15 minutes; quenched with water and 20 mL 99:1 (v/v) hexane-triethylamine. The organic layer was separated, washed with 3 x 20 mL saturated brine and dried over anhydrous sodium sulfate. The aqueous layers were back extracted with 50 mL 99:1 (v/v) hexane-triethylamine; the combined, dried extracts were evaporated *in-vacuo* to a small volume and chromatographed on silica gel using n-hexane-ether-triethylamine (89:10:1 v/v/v) as eluent. The fractions containing the title compound were combined and evaporated *in-vacuo* to give the title compound (64%) as a colorless oil. This compound was dissolved in n-hexane (containing 1% triethylamine) and stored under argon (-50° C) until used in further transformations.

Analytical Data : UV (cyclohexane): $\lambda_{274}$(sh) ($\epsilon$ 36,200), $\lambda_{282}$ ($\epsilon$ 44,700), $\lambda_{295}$(sh) ($\epsilon$ 32,000); NMR (cyclohexane - $d_{12}$)$\delta$: 0.9 (3 H, t), 1.1-2.3 (17 H, m), 2.9 (2 H, t, J=6.3 Hz), 3.05 (1 H, d, J=6.8 Hz), 3.5 (3 H, s), 5.1-6.6 (m, 8 vinyl protons); CMR (cyclohexane-$d_{12}$) $\delta$: 172.4, 135.1, 132.3, 131.2, 131.0, 129.1, 128.8, 127.7, 62.7, 50.8, 38.4, 33.9, 32.3, 30.1, 21.2, 16.7, 14.3; IR ($CHCL_3$): 2945, 2920, 1730, 1440, 995, 975 $cm^{-1}$; MS m/e: 347 ($M^+$ + 1), 346 ($M^+$), 329 (347 - $H_2O$), 328 (346 - $H_2O$), 315 (346 -$OCH_3$), 235 (346 - $C_8H_{15}$), 91 (base peak).

Example 65 :

5S-Hydroxy-5-methyl-6R-(4-carboxyphenylthio)eicosa-7E,9E,11Z,14Z-tetraenoic Acid (MLS-7356)

Step a

Preparation of Methyl 5S-Hydroxy-5-methyl-6R-(4-carbomethoxyphenylthio)eicosa-7E,9E,11Z,14Z-tetraenoate

To a solution, in the dark, under argon, containing 11 mg of methyl 5-methyl-5S,6S-epoxyeicosa-7E,9E,-11Z,14Z-tetraenoate (Example 64) and 0.12 mL N,N-diethylcyclohexylamine in 0.12 mL methanol was added, 39 mg of methyl 4-mercaptobenzoate. After 17 hours, the reaction mixture was diluted with 1.0 mL of 87:13 (v/v) acetonitrile-water and the mixture purified by semipreparative HPLC (2 x waters, C-18, $\mu$-Bondapake® columns, 87:13 (v/v) acetonitrile-water as eluant. In this manner the title compound was obtained as a white solid.

Analytical Data : UV (methanol): $\lambda_{279}$ ($\epsilon$ 56,300), $\lambda_{295}$(sh) ($\epsilon$ 34,800); $R_v$ 6.25 (87:13 (v/v) acetonitrile-water, 5$\mu$, C-18, ASI analytical column).

Step b

5S-Hydroxy-5-methyl-6R-(4-carboxyphenylthio)eicosa-7E,9E,11Z,14Z-tetraenoic Acid

The solid dimethyl ester (21 d, 3.9 mg) from step a above was stirred in the dark under argon in a solution containing 0.6 M (0.125 mL) lithium hydroxide and 1.0 mL methanol until hydrolysis was complete (15.5 hours). The reaction solution was evaporated *in-vacuo* and diluted with 5 mL water containing 100 mg sodium dihydrogenphosphate monohydrate. This solution was desalted using Waters C-18 Sep-Pak® using methanol as final eluant. After evaporation of solvent *in-vacuo* , the title compound was obtained as a white solid.

Analytical Data : UV (methanol): $\lambda_{278}$ ($\epsilon$ 42,100), $\lambda_{295}$(sh) ($\epsilon$ 27,800); $R_v$ 2.99 (65:35 (v/v) pH 7.2 phosphate buffer-acetonitrile, 5$\mu$, C-18, ASI analytical column).

Many modifications and variations can be made without departing from the spirit and scope of the invention which is solely defined by the claims following.

## Claims

1. A leukotriene antagonist, comprising compound I, esters, amides and physiologically acceptable salts thereof, where compound I is represented by the formula

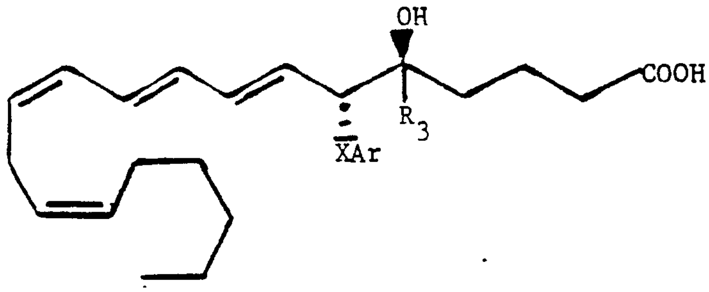

I

wherein $R_3$ is H or a lower alkyl group of 1 to 6 carbon atoms; and XAr is selected from

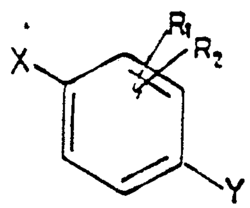

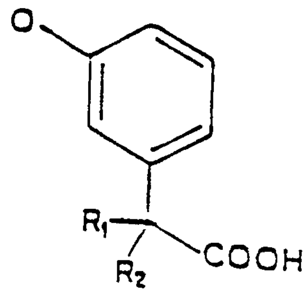

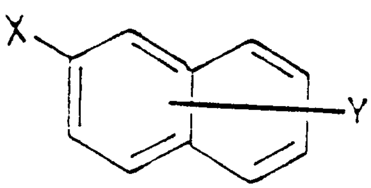

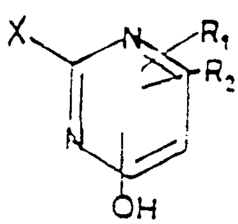

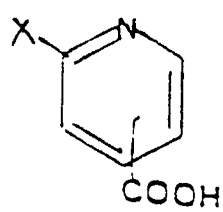

where X is selected from S,O. SO, $SO_2$ or $SCH_2$;
$R_1$ and $R_2$ are, same or different, H, lower alkyl, lower alkoxy, alkoxyalkyl, or $C_6H_4COOH$; and
Y is selected from H, OH, $CO_2H$, $CHN_4$, $CONH_2$, $CO_2R_1$, $OR_1$, $CH(CO_2H_2)_2$, $CR_1R_2CO_2H$ or $C_6H_4COOH$; with the proviso that when $R_3$ is hydrogen, XAr is not

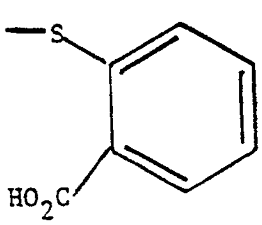

or

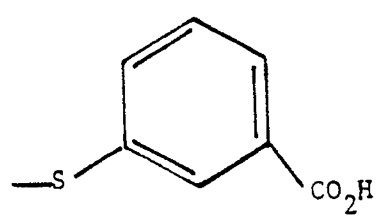

2. The compounds of claim 1 wherein Y is H and $R_1$ is $C_6H_4COOH$ and $R_2$ is H, lower alkyl, lower alkoxy or an alkoxyalkyl group.

3. The compound of claim 1 wherein $R_3$ is H and XAr is selected from

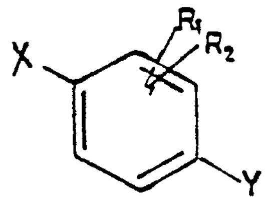

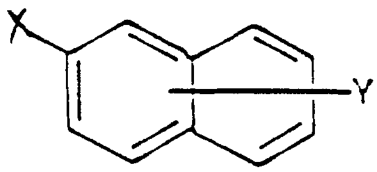

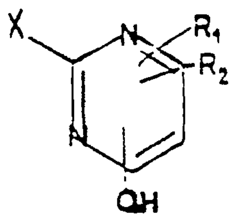

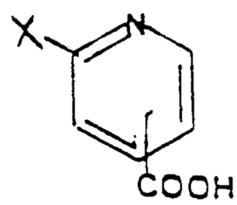

where $R_1$ and $R_2$, are the same or different, H, lower alkyl, or alkoxyalkyl; X is selected from -O-, -S-, or $-SH_2-$; and

Y is selected from OH, $CO_2H$, $CHN_4$, $CONH_2$, $CO_2R_1$, $OR_1$, $CH(CO_2H_2)_2$, $CR_1R_2CO_2H$.

4. The compound of claim 3 wherein XAr is selected from

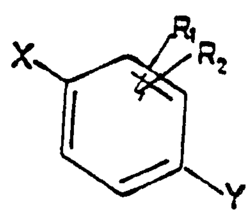

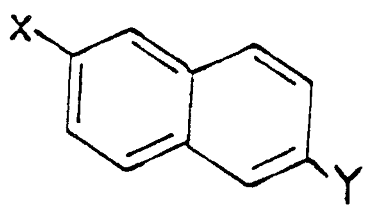

wherein $R_1$ and $R_2$, are same or different, H, lower alkly or alkoxyalkyl; and

Y is selected from OH, $CO_2H$, $CHN_4$, $CONH_2$, $CO_2R_1$, $OR_1$, $CH(CO_2H_2)_2$, $CR_1R_2CO_2H$.

5. The compound of claim 3 wherein -XAr is

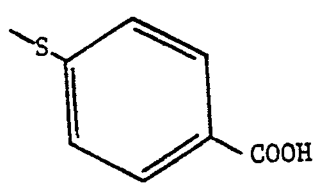

6. The compound of claim 3 wherein -XAr is

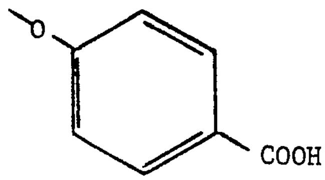

7. A leukotriene antagonist, comprising compound II, esters and physiologically acceptable salts thereof, where compound II is represented by the formula

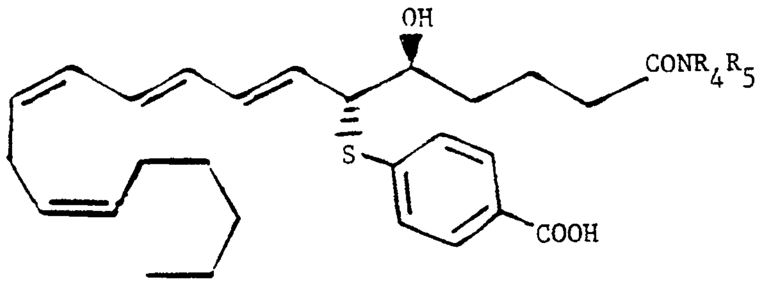

II

wherein $R_4$ and $R_5$, same or different are selected from H, alkyl or aryl, optionally substituted by one or more carboxy or amino groups, or $R_4$ and $R_5$ together with the nitrogen form a heterocyclic ring.

8. A process for the preparation of leukotriene antagonists, esters, amides or physiologically acceptable salts thereof according to claims 1 to 7, characterized in that leukotriene A4 derivatives of the formula

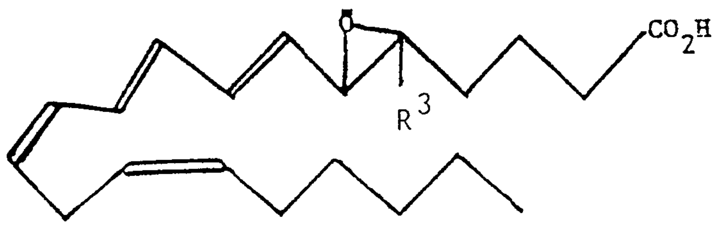

$LTA_4$

are reacted with mercaptansor alcohols of the formula

HX′Ar

where X denotes -S- or -O-

in suitable solvents in the presence of trialkylamine bases, then the ester produced is optionally hydrolyzed, optionally oxidized (X = SO, $SO_2$) and optionally transferred into amides or into their physiologically acceptable salts.

9. Medicaments containing leukotriene antagonists according to claims 1 to 7.

10. Use of leukotriene antagonists according to claims 1 to 7 for the preparation of medicaments.

European Patent Office

# EUROPEAN SEARCH REPORT

Application Number

EP 90113516.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 98, no. 7, February 14, 1983 Columbus, Ohio, USA ONO PHARMACEUTICAL "Leukotriene - related compounds" page 639, column 2, page 640, column 1, abstract-no. 53 540f & JP-A-57 118 555 -- | 1,8 | C 07 C 59/68<br>C 07 C 69/736<br>C 07 C 317/46<br>C 07 C 323/54<br>C 07 C 235/28<br>A 61 K 31/20<br>A 61 K 31/22<br>A 61 K 31/16 |
| A | CHEMICAL ABSTRACTS, vol. 103, no. 21, November 25, 1985 Columbus, Ohio, USA P.R. BERNSTEIN et al. "Preparation of diketopiperazine analog of leukotriene D4(LTD4)" page 659, column 2, abstract -no. 178 059j & Tetrahedron Lett. 1985, 26(16), 1951-4 -- | 1 | TECHNICAL FIELDS SEARCHED (Int. Cl.5) |
| D,X | EP - A2 - 0 147 217 (MERCK FROSST) * Claims * -- | 1-10 | C 07 C 59/00<br>C 07 C 69/00<br>C 07 C 235/00<br>C 07 C 323/00<br>A 61 K 31/00 |
| D,A | US - A - 4 513 005 (ST.R. BAKER et al.) * Claims * ---- | 1,9,10 | |

The present search report has been drawn up for all claims

| Place of search | Date of completion of the search | Examiner |
|---|---|---|
| VIENNA | 15-10-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document